# EUROPEAN PATENT APPLICATION

(11) **EP 3 392 339 A1**
(43) Date of publication of application: **24.10.2018**
(21) Application number: 17166753.8
(22) Date of filing: 18.04.2017
(51) Int. Cl.: C12N 15/82

(54) **IMPROVED GENOME EDITING IN PLANT CELLS**

(71) Applicant: Rheinisch-Westfälische Technische Hochschule (RWTH) Aachen, 52062 Aachen (DE)
(72) Inventor: Bortesi, Luisa, 52066 Aachen (DE); Sruthy, Maria, 52070 Aachen (DE); Fischer, Rainer, 52076 Aachen (DE); Sack, Markus, 52477 Alsdorf (DE); Zischewski, Julia, 52074 Aachen (DE)
(74) Representative: Roth, Andy Stefan

(57) **Abstract**

The technology provided herein relates to methods for the delivery of pre-assembled ribonucleoprotein complexes (RNP) - such as the Cas9/gRNA RNP complex - for highly efficient genome editing to intact differentiated cells and regeneration of cell lines, whole tissues or organisms thereof.

## Description

### FIELD OF THE DISCLOSURE

The technology provided herein relates to methods for the delivery of pre-assembled ribonucleoprotein complexes (RNP) - such as the Cas9/gRNA RNP complex - for highly efficient genome editing to intact differentiated cells and regeneration of cell lines, whole tissues or organisms thereof.

### BACKGROUND

The variety of traits found in nature reflects the presence of one or multiple mutations in certain genes, which often arise as mistakes in the repair mechanism initiated after DNA damage. To expand the repertoire of traits readily available for breeding, methods for artificially changing genomes by means of mutations induced by chemicals and radiation have been developed. More recently, strategies such as tilling (Targeting Induced Local Lesions in Genomes)have been developed as reverse genetics tools, which also entail the use of a chemical or radiation mutagens, causing extensive DNA damage and therefore, mutations. These classical approach considerably expanded the range of practically useful traits available for breeding and accelerated the establishment and release of improved plant varieties. However, as the mutations are induced randomly, extensive screening efforts to identify the mutation of interest are still needed. In addition, as many mutations are induced simultaneously, after successful identification of a line displaying the desired trait, several rounds of backcrossing are needed to eliminate most of the background mutations. With the discovery of site-directed nucleases (SDNs) and the advent of genome editing it is now possible to induce a break, and therefore a mutation, at specific pre-determined locations in the genome. These locations in the genome are defined by their sequence and the sequence specificity of the DNA-modifying enzymes. In fact, when a double strand break occurs in a higher eukaryotic cell, it is often repaired via the Non-Homologous End Joining (NHEJ) pathway resulting in small insertions or deletions (indels) which mutate the targeted gene. If a short piece of double or single stranded DNA homologous to the targeted region but carrying a single or few base mutations is provided along with the nuclease, the break can be repaired by the Homology-Directed Repair (HDR) pathway, allowing to precisely edit a few nucleotides in the target gene.

The latest ground-breaking genome editing technology is based on RNA-guided engineered nucleases, which have rapidly set themselves as a very efficient and versatile tool. Currently, the most widely used system is the type II clustered regularly interspaced short palindromic repeat (CRISPR)/Cas9 (CRISPR-associated) system from Streptococcus pyogenes. The natural system comprises small interfering CRISPR RNAs (crRNAs) approximately 40 nt in length, which combine with the transactivating CRISPR RNA (tracrRNA) to activate and guide the Cas9 nuclease. This cleaves homologous double-stranded DNA sequences known as protospacers in the invading DNA. A prerequisite for cleavage is the presence of a conserved protospacer-adjacent motif (PAM) downstream of the target DNA, which usually has the sequence 5'-NGG-3'. The target DNA sequence can be reprogrammed simply by changing 20 nucleotides in the crRNA and the targeting specificity of the crRNA can be combined with the structural properties of the tracrRNA in a chimeric single guide RNA (gRNA) (Jinek et al., 2012).

Since their first discovery, SDNs including the CRISPR/Cas9 components (Cas9 and single guide RNA) have been introduced into plants as DNA (plasmid, dsDNA fragment or single-stranded T-DNA), generating de facto transgenic plants or transgenic intermediates (when the transgene is segregated out or eliminated in a subsequent step). However, production of transgenic plants is a very controversial topic, with public opinion often being against the creation of such varieties, particularly for crop plants that will be grown over large geographical areas and used as food for human consumption. Even in the case where the transgene is segregated out via crossing or is delivered as a plasmid or viral replicon and expressed only transiently in the plant cell, it is possible that the DNA or small fragments thereof are inserted at random position in the genome. These can be detected with certainty only by whole genome sequencing to be able to ultimately determine if the resulting plants falls into the transgenic category (Kim and Kim 2016). However, whole genome sequencing is a rather expensive and time-consuming procedure and, most importantly, it cannot be performed for species for which a reference genome is not available in the first place. Moreover, the question whether or not a genome modified plant has to be considered as transgenic can depend on whether or not recombinant DNA has been used in the process rather than whether this recombinant DNA is comprised in the final organism.

Direct delivery of RNP complexes for genome editing is highly desirable because it does not involve DNA, precluding the possibility of the resulting plant to be considered transgenic. The problem of delivering RNP complexes into plant cells is that they have a cell wall which hinders the entry of large molecules inside the cell. Gene editing by direct delivery of the CRISPR RNP complex into protoplasts via PEG transfection and regeneration of plants thereof has been described (Woo et al., 2015).

However, obtaining protoplasts from plant tissues and then regenerating whole plants form single protoplasts is a very delicate procedure that has to be optimized for each plant species (in some cases even cultivar), and it is well established only for a handful of species. Notably, many important crops cannot be regenerated from protoplasts efficiently.

In contrast, regeneration of whole plants from several tissues is a more straightforward procedure for many more species, including some of major economic relevance. Direct delivery of RNP complexes into maize embryo cells and bread wheat embryo cells by particle bombardment and regeneration of plants thereof has also been reported (Svitashev et al., 2016; Liang et al., 2017). Yet, in many cases only particular cultivars are performing well in tissue culture, thus making subsequent breeding into agronomical important cultivars necessary. Tissue culture and particularly the generation of dedifferentiated cells often induces somaclonal variations and epigenetic effects, hence methods reducing or avoiding these steps are also highly desirable.

However, the availability of simpler and more effective ways of delivering ribonucleoprotein (RNP)-complexes into intact cells, in particular into differentiated plant cells with a cell wall of a wide variety of plant species is needed.

### SUMMARY OF THE DISCLOSURE

The present disclosure pertains to methods for the delivery of pre-assembled ribonucleoprotein complexes (RNP) - such as the Cas9/gRNA RNP complex-for highly efficient genome editing to intact differentiated cells and regeneration of cell lines, whole tissues or organisms thereof.

Moreover, the novel approach of the present disclosure reduces or even eliminates the dependence on sterile tissue culture and thereby make a larger variety of species amenable for genome-editing in a faster and more cost- and time effective manner.

In particular, with the methods according to the present disclosure pre-assembled RNP complexes are either directly delivered through the cell wall into the cell (by biolistic delivery) or the passive uptake of the RNP complex is facilitated by creating local lesions in the cell wall by physical delivery methods including mechanical methods like abrasion (e.g. with silicon carbide or whiskers) or perforation (e.g. with echo waves or laser). Because the protein used has repetitive nuclear localization signals, once the pre-formed RNP complexes are inside the cell they are readily transported into the nucleus where they can cleave the DNA at the target site. This is a major difference compared to the transfer of nucleic acids usually performed with the above-mentioned techniques, and it most likely accounts for the exceptionally high mutation rates observed when delivering the RNP complex directly. With the methods described in the present disclosure, the genome-editing efficiencies are so high that it is possible to regenerate and recover whole plants with the desired mutation without applying any kind of selection, e.g. by using an antibiotic or herbicide resistance gene or an enzyme that enables colorimetric detection or a fluorescent protein that facilitates non-invasive fluorometric detection.

In a first aspect, embodiments of the disclosure provide novel methods for altering the genome of an intact differentiated cell(s) without inserting exogenous genetic material into the genome comprising:
(i) providing an intact differentiated cell that comprises an endogenous gene to be modified;
(ii) providing a genome-modifying formulation comprising a pre-assembled ribonucleoprotein (RNP)-complex comprising a nucleic acid modifying protein and a ribonucleic acid;
(iii) delivering said RNP-complex into the cell by using a physical delivery method;
(iv) inducing one or more single or double stranded DNA breaks in the cell genome to produce a cell having a detectable targeted genomic modification without the presence of any exogenous genetic material in the cell genome. In a second aspect, embodiments of this disclosure relate to methods for manufacturing particles comprising a ribonucleoprotein (RNP)-complex suitable for delivering a ribonucleoprotein (RNP)-complex into an intact differentiated cell, comprising the steps of:
   a) *in vitro* assembly of a RNP-complex comprising a nucleic acid modifying protein and a ribonucleic acid;
   b) coating a particle with the pre-assembled RNP-complex;
   c) loading the coated particle onto a macrocarrier.

In a third aspect, embodiments of this disclosure relate to methods for delivering a ribonucleoprotein (RNP)-complex into intact differentiated cells comprising the steps of:
(i) providing an intact differentiated cell;
(ii) providing a pre-assembled ribonucleoprotein (RNP)-complex comprising a nucleic acid modifying protein and a ribonucleic acid;
(iii) delivering of said RNP-complex into the cell with by using a physical delivery method.

In a fourth aspect, embodiments of this disclosure provide methods for delivering a ribonucleoprotein (RNP)-complex into intact differentiated cells comprising the steps of:
(i) providing an intact differentiated cell;
(ii) providing a pre-assembled ribonucleoprotein (RNP)-complex comprising a nucleic acid modifying protein and a ribonucleic acid;
(iii) Bringing the pre-assembled ribonucleoprotein (RNP)-complex in contact with the cell by using a carrier material, resulting in
(iv) uptake and/or delivery of the RNP into the cell and
(v) alteration/modification of the genome of the cell.

Before the disclosure is described in detail, it is to be understood that this disclosure is not limited to the particular component parts of the process steps of the methods described. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

### BRIEF DESCRIPTION OF THE DRAWINGS

Figure 1 shows A) DsRed fluorescence in *N.benthamiana* leaves (20X magnification) after single bombardment with 1 µm gold particle at 650 psi helium pressure with 6 cm target distance. The amount of DsRed used in the experiment were: A) 10 µg B) 5 µg C) 2 µg D) 300 ng E) 200 ng F) 100 ng G) 50 ng H) negative control with untreated gold particles I) wild-type. Images H and I were taken at lower magnification (5X magnification) and B) Table 1 as a summary of proteolistic delivery of DsRed in *N.benthamiana* leaves.
Figure 2 shows A) DsRed fluorescence in *Nicotiana tabacum* cv. BY2 suspension cells (20X magnification) after single bombardment with 1 µm gold particle at 650 psi helium pressure with 6 cm target distance. The amount of DsRed used in the experiment were: A) 300 ng B) 200 ng C) 100 ng D) 50 ng E) negative control with untreated gold particles F) wild-type. Images E and F were taken at lower magnification (5X magnification) and B) Table 2 as a summary of proteolistic delivery of DsRed in BY2 cells.

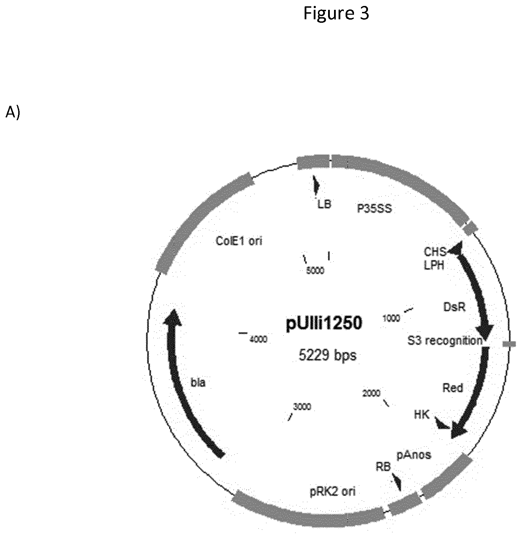

Figure 3 is a map of pUlli1250- a) The vector backbone is from pPAM (GenBank: AY027531.1). The expression cassette for the SSA reporter consists of the CaMV 35S promoter with duplicated enhancer region (P35SS); the 5' UTR of the chalcone synthase gene from *Petroselinum crispum* (*CHS*); the codon-optimized murine signal peptide of mAb24 (*LPH*); the split coding sequence of DsRed with direct repeats (DsR and *Red,* respectively); the CRISPR/Cas9 target site (*S3 recognition*); a 6x-histidine tag and a SEKDEL tag for ER-localization (HK); the terminator of the nopaline synthase gene of *A. tumefaciens* (pAnos).
Figure 4 is a) a schematic representation of the T-DNA region of the positive control plasmid pUlli1244. Cas9 is under the control of an enhanced 35S promoter and the NOS terminator, while the gRNA is under the control of the Arabidopsis U6 promoter and terminator. b) Fluorescence microscope image taken 5 days post infiltration (dpi).
Figure 5 is a) a schematic representation of the T-DNA region of the negative control plasmid pUlli1232. b) Fluorescent microscope image taken 5 days post infiltration (dpi).
Figure 6 is a) a schematic representation of the T-DNA region of the negative control plasmid pUlli541. b) Fluorescence microscope image at 5 dpi.
Figure 7 shows a) a schematic representation of the T-DNA regions of the DsRed SSA plasmid pUlli1250, and b) a fluorescence microscope image 5 days post infiltration (dpi).
Figure 8 shows A) DsRed fluorescence observed in *N. benthamiana* leaves at the fluorescent microscope (10x) after agro-infiltration of the SSA construct followed by bombardment with the RNP complex a) 50 µg b) 25 µg Cas9 c) 10 µg Cas9 d) 1 µg Cas9 and e) untreated gold particles bombarded on infiltrated leaf and f) WT and B) Table 4 as a summary of DsRed intensity in *N. benthamiana* leaves.
Figure 9 shows A) DsRed fluorescence in BY2 cells transiently expressing the SSA reporter construct pUlli1250 after bombardment with RNP complex. a) 50 µg b) 25 µg Cas9 c) 10 µg Cas9 d) untreated gold particles bombarded on infiltrated BY2 cells e) non-infiltrated and non-bombarded BY2 cells and B) Table 5 as a summary of DsRed intensity in BY2 cells.
Figure 10 shows an agarose gel showing the results of a T7 endonuclease 1 assay confirming genome editing activity of RNPs. The expected bands are indicated with white arrows.
Figure 11 shows an agarose gel showing the results of a T7 endonuclease 1 assay confirming genome editing activity of RNPs. The expected bands are indicated with white arrows.
Figure 12 shows an agarose gel showing the results of a T7 endonuclease 1 assay confirming genome editing activity of RNPs. The expected bands are indicated with white arrows.
Figure 13 shows an agarose gel showing the results of a T7 endonuclease 1 assay confirming genome editing activity of RNPs. The expected bands are indicated with white arrows.
Figure 14 shows an agarose gel showing the results of a T7 endonuclease 1 assay confirming genome editing activity of RNPs. The expected bands are indicated with the arrows.
Figure 15 shows DsRed fluorescence in *N.benthamiana* leaves (20 x magnification) after abrasion with Celite-503. The amount of DsRed used in the experiment were: A) 2 µg B) 5 µg C) 10 µg D) negative control (MilliQ water only) E) wild-type.
Figure 16 shows an agarose gel showing the results of a T7 endonuclease 1 assay confirming genome editing activity of RNPs. The expected bands are indicated with white arrows.
Figure 17 shows an agarose gel showing the results of a T7 endonuclease 1 assay confirming genome editing activity of RNPs. The expected bands are indicated with white arrows.
Figure 18 shows an agarose gel showing the results of a T7 endonuclease 1 assay confirming genome editing activity of RNPs. The expected bands are indicated with white arrows.

### DETAILED DESCRIPTION OF THE DISCLOSURE

The present disclosure pertains to the delivery of pre-assembled ribonucleoprotein complexes (RNP) - such as the Cas9/gRNA RNP complex - for highly efficient genome editing of intact plant cells and regeneration of whole plants thereof. The RNP complex is allowed to form *in vitro* and then is delivered through the plant cell wall by various physical delivery methods including mechanical methods, such as particle bombardment, surface abrasion with silicon carbide powder or whiskers, perforation with echo waves or a laser.

The "CRISPR/Cas" system refers to a widespread class of bacterial systems for defense against foreign nucleic acid. CRISPR/Cas systems are found in a wide range of eubacterial and archaeal organisms. CRISPR/Cas systems include type I, II, and III sub-types. Wild-type type II CRISPR/Cas systems utilize an RNA-mediated nuclease, Cas9 in complex with guide and activating RNA to recognize and cleave foreign nucleic acid. Guide RNAs having the activity of both a guide RNA and an activating RNA are also known in the art. In some cases, such dual activity guide RNAs are referred to as a small guide RNA (sgRNA).

By delivering the RNP as a pre-formed complex, the editing efficiency is so high that whole plants carrying mutations at the desired target site can be regenerated and recovered without applying any selection. In contrast to the described procedure of delivering the RNP complex by protoplast transfection, with the present methods eukaryotic cells like plant cells (i.e. suspension cells, calli, leaves, stems, cotyledons, pollen, etc.) can be readily edited without the need of going through the protoplast stage. As not all plant species are amenable to protoplasting, and regeneration of whole plants from protoplasts is technically difficult, this is a major improvement that greatly simplifies and most importantly extends the use of DNA-free genome editing to basically any plant species that can regenerate from tissue culture.

DNA-free genome editing is highly desirable as it may be exempt from GMO regulations and the ability to use proteins or pre-assembled ribonucleoprotein complexes (RNP) facilitates an entire range of application possibilities.

In one aspect, the present disclosure provides methods for altering the genome of an intact differentiated cell (e.g. altering or modifying expression of one or more gene products).

In fact, these methods have been traditionally used to deliver nucleic acids in association with a selection agent to identify and/or recover the events of interest, or have been used to introduce self-replicating viruses or viral replicons that only require a minimal starting inoculum to infect a whole plant. Indeed, when biolistic delivery of a fluorescent protein into intact plant tissues was investigated by the inventors (Examples 0A and 0B) and others (Martin-Ortigoza et al., 2014), only a few spots of fluorescence where visible within the bombarded tissue area. In contrast, when we used the biolistics to deliver the Cas9/crRNA/tracrRNA RNP complex into tissues expressing the DsRed SSA reporter, an unexpectedly homogenous fluorescence comprising almost the whole tissue was observed.

Compared to delivery to protoplasts, the present invention greatly simplifies the procedure to obtain genome edited plants because all the steps necessary to obtain and regenerate the protoplasts are not necessary. In addition, the present invention extends the procedure of RNP complex delivery to many more plant species which are not amenable to regenerate from protoplasts, i.e. to basically any plant species that can regenerate from tissue culture. The present invention facilitates genome-editing to more plant species, in a faster and more efficient manner and represents a significant competitive advantage for generating and breeding the next generations of crops and designer-plants for various purposes.

In one aspect, the RNP complex is first allowed to assemble *in vitro,* mixed with gold particles and directly loaded onto the macrocarrier, where it is simply let air dry, and then delivered into the cells by bombardment. The RNP complex can be constituted e.g. of a Cas9 protein and a single guide RNA or the Cas9 protein, the guide RNA and the tracrRNA. The complex can be constituted by any other nuclease associated with the RNA determining the target specificity. To those skilled in the art it will be apparent that the RNP can be also constituted of a nuclease with a modified RNA molecules such as, but not limited to, a RNA molecule carrying base analogue substitutions or base modifications or substitutions of the phosphodiester bond.

In another aspect, the RNP complex is first allowed to assemble *in vitro* and is then introduced into differentiated leaf cells by applying the formulation comprising the RNP complex onto the leaf surface and abrading the leaf surface with silica powder (celite). Unlike nanoparticle mediated delivery (US 9187755 B2; Nanoparticle mediated delivery of sequence specific nucleases) this method is much simpler because it does not require to coat a nanoparticle by noncovalent adsorption of the RNP complex on the surface of the nanoparticle or into the nanoparticle.

In yet another embodiment, the DNA modifying protein and the nucleic acid(s) or nucleic acid analog(s) that are not DNA are delivered separately into the plant cell.

The plant cells comprising a cell wall can be obtained from cell suspensions of single cells or cell aggregates or from tissues such as anthers, callus, cotyledons, embryo, flowers, leaves, pods, roots, seeds and stems or can be part of an intact plant.

The RNP complex is not restricted to nuclease activity. For example, a catalytically inactive version of the Cas9 protein is unable to cut DNA but it can still be recruited to specific DNA sequences by gRNAs. If it is expressed as a fusion protein with, e.g., a transcriptional activator or a cytidine deaminase, it can be delivered as RNP complex to e.g. activate transcription of a specific gene or mediate the direct conversion of cytidine to uridine.

The methods described in the present disclosure may be used to deliver other DNA-modifying enzymes including, but not limited to, Zinc Finger Nucleases (ZFN), Transcription Activator-Like Effectors (TALE), TALE nucleases (TALEN), methyl-transferases, histone deacethylases transcription factors and transcription repressors.

As used in the present disclosure, "cell", "cell line", and "cell culture" can be used interchangeably and all such designations include progeny. It is also understood that all progeny may not be precisely identical in DNA content, due to deliberate or inadvertent mutations. Mutant progeny that have the same functionality as screened for in the originally transformed cell are included.

As used herein, the phrase "coding sequence", "encoding sequence", "structural nucleotide sequence" or "structural nucleic acid molecule" refers to a nucleotide sequence that is translated into a polypeptide, usually via mRNA, when placed under the control of appropriate regulatory sequences. The boundaries of the coding sequence are determined by a translation start codon at the 5'-termmus and a translation stop codon at the 3'-terminus. A coding sequence can include, but is not limited to, genomic DNA, cDNA, EST and recombinant nucleotide sequences.

The term "gene" refers to a DNA sequence that comprises control and coding sequences necessary for the production of a recoverable bioactive polypeptide or precursor. Endogenous gene are those that originate from within an organism, tissue, or cell.
The term "isolated" describes any molecule separated from its natural source.

As used herein, the term "nucleic acid" refers to a single or double-stranded polymer of deoxyribonucleotide or ribonucleotide bases read from the 5' to the 3' end. The "nucleic acid" may also optionally contain non-naturally occurring or altered nucleotide bases that permit correct read through by a polymerase and do not reduce expression of a polypeptide encoded by that nucleic acid. The term "nucleotide sequence" or "nucleic acid sequence" refers to both the sense and antisense strands of a nucleic acid as either individual single strands or in the duplex. The term "ribonucleic acid" (RNA) is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA) and the term "deoxyribonucleic acid" (DNA) is inclusive of cDNA and genomic DNA and DNA-RNA hybrids. The words "nucleic acid segment", "nucleotide sequence segment", or more generally "segment" will be understood by those in the art as a functional term that includes both genomic sequences, ribosomal RNA sequences, transfer RNA sequences, messenger RNA sequences, operon sequences and smaller engineered nucleotide sequences that express or may be adapted to express, proteins, polypeptides or peptides.

As used herein, the term "Intact cells" are characterized by an intact cell membrane and/or cell wall. In particular, if the intact cells are eukaryotic cells like plant cells, and the plant cells comprise an intact cell wall. Preferably, an intact cell is collected from and/or comprised within a differentiated tissue.

As used in this disclosure "differentiated" and "undifferentiated" are relative terms depending on the context in which they are used. Specifically, in reference to a particular type of self-renewing stem cell, the term "undifferentiated" refers back to the same self-renewing stem cell, whereas the term "differentiated" refers to one or more of the relatively mature phenotypes the stem cell can generate - as discernable by morphological criteria, antigenic markers, and gene transcripts they produce. Undifferentiated pluripotent stem cells including iPS cells have the ability to differentiate into all three germ layers. The cells differentiated from them do not, and can readily be recognized by one skilled in the art by morphological criteria. In particular, differentiated plant cells include but are not limited to epidermal and mesophyll cells from cotyledons or leaves, epidermal or vascular cells from roots, epidermal or vascular cells from stems.

The term "altering the genome" is used in the present disclosure for inducing one or more single or double stranded DNA breaks (DSB) in the cell genome, which results in a mutation that lead to / result in inhibiting expression of a target gene in said cell of which refers to the absence (or observable decrease) in the level of protein and/or mRNA product from the target gene.

The term "plant" includes the plant body, plant organs (for example, leaves, petals, stem, root, rhizome, and seeds), plant tissues (for example, epidermis, phloem, parenchyma, xylem, and vascular bundle), and plant cells. In addition, the term "plant cell" includes cell suspension cultures, embryos, meristematic tissue regions, callus tissues, cells derived from leaves and roots. When plant culture cells are transformed, an organ or individual is regenerated from the transformed cells by a known tissue culture method. These operations are readily performed by those skilled in the art. An example is described below. Firstly, the transformed plant cells are cultured in a sterilized callus forming medium (containing a carbon source, saccharides, nutrients, vitamins, inorganics, and phytohormones such as auxin and cytokinin), thereby forming a dedifferentiated callus which indefinitely proliferates (callus induction). The formed callus is transferred to a new medium containing a plant growth regulator such as auxin, and further proliferated thereon (subcultivation). When the callus induction is carried out on a solid medium such as agar and subcultivation is carried out in a liquid medium, the respective cultures are efficiently achieved. Secondly, the callus proliferated by subcultivation is cultured under appropriate conditions, thereby inducing re-differentiation of the organ (inductive re-differentiation), and regenerating the plant body. The inductive re-differentiation is achieved by appropriately adjusting the type and amount of the various components of the medium, including plant growth regulators such as auxin and cytokinin, and the carbon source, and the light and temperature. The inductive re-differentiation forms adventitious embryos, adventitious roots, adventitious buds, adventitious foliage, and others, and they are grown into a complete plant body. The plant before being a complete plant body may be stored in the form of, for example, capsulated artificial seeds, dry embryos, lyophilized cells, or tissues.

As used herein and mentioned above "genetic modification" or "altering the genome" means that a gene may be removed, or "knocked out", using a nucleic acid modifying protein like a nuclease. The present disclosure pertains to methods for editing eukaryotic genomes like mammalian or plant genomes using non- transgenic strategies. Sequence-specific nucleases (including ZFNs, homing endonucleases, TAL-effector nucleases, CRISPR-associated systems [Cas9]) are introduced into plant cells in the form of a pre-assembled ribonucleoprotein (RNP)-complex comprising a nucleic acid modifying protein and a ribonucleic acid. The functional nucleases are targeted to specific sequences, and cut the cellular DNA at predetermined loci. The DNA damage triggers the plant cell to repair the double strand break. Mistakes (e.g., point mutations or small insertions/deletions) made during DNA repair then alter DNA sequences in vivo. As mentioned above, unlike conventional DNA transformation, the protein or RNA-based genome editing strategies described herein specifically modify target nucleic acid sequences at a high rate, i.e. the vast majority of mutations are found in the genome location defined by the sequence-specificity of the RNP.. Since no foreign DNA is used in these methods, this process is considered to be non-transgenic plant genome editing.

The nucleic acid modifying protein may be a sequence -specific nuclease like a TAL effector- nuclease, a homing endonuclease, a zinc finger nuclease (ZFN), or a CRISPR enzyme like a CRISPR-Cas9 endonuclease. The term "CRISPR enzymes", such as a Cas protein, include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. These enzymes are known; for example, the amino acid sequence of *S*. *pyogenes* Cas9 protein may be found in the SwissProt database under accession number Q99ZW2. In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some advantageous embodiments, the nucleic acid modifying protein is a CRISPR-Cas9 endonuclease, a CRISPR-Cpf1 nuclease or a CRISPR-C2c2 endoribonuclease.

In some advantageous embodiments, the RNP-complex used in the methods of the present disclosure comprises a nucleic acid comprising a crRNA and a tracrRNA, or a chimeric cr/tracrRNA hybrid, wherein the crRNA and tracrRNA, or the cr/tracrRNA hybrid, is targeted to a sequence that is endogenous to the cell; and a Cas9 endonuclease molecule that induces a double strand break at or near the sequence to which the crRNA and tracrRNA sequence is targeted, or at or near the sequence to which the cr/tracrRNA hybrid is targeted.

As used herein the term "endonuclease" refers to an enzyme capable of causing a single or double - stranded break in a DNA molecule.

As defined herein the term "exonuclease" refers to an enzyme that works by cleaving nucleotides one at a time from the end (exo) of a polynucleotide chain causing a hydrolyzing reaction that breaks phosphodiester bonds at either the 3' or the 5 ' to occur.

As used herein the term "sequence-specific nuclease" refers to any nuclease enzyme which is able to induce a double-strand DNA break at a desired and predetermined genomic locus

As used herein the term "meganuclease" refers to natural or engineered rare- cutting endonuclease, typically having a polynucleotide recognition site of about 12- 40 bp in length, more preferably of 14-40 bp. Typical meganucleases cause cleavage inside their recognition site, leaving 4 nt staggered cut with 3'OH overhangs. The meganuclease are preferably homing endonuclease, more particularly belonging to the dodecapeptide family (LAGLIDADG; SEQ ID NO: 55) (WO 2004/067736), TAL- effector like endonuclease , zinc-finger-nuclease, or any nuclease fused to modular base-per-base binding domains (MBBBD) - i.e., endonucleases able to bind a predetermined nucleic acid target sequence and to induce cleavage in sequence adjacent thereto. These meganucleases are useful for inducing double-stranded breaks in specific DNA sequences and thereby promote site-specific homologous recombination and targeted manipulation of genomic sequences.

As used herein the term "vector" refers to a nucleic acid molecule capable of transporting another nucleic acid to which it has been linked into a cell, or a cell compartment.

As used herein the term "zinc finger nuclease" refers to artificial restriction enzymes generated by fusing a zinc finger DNA-binding domain to a DNA-cleavage domain. Briefly, ZFNs are synthetic proteins comprising an engineered zinc finger DNA-binding domain fused to the cleavage domain of the Fokl restriction endonuclease. ZFNs may be used to induce double -stranded breaks in specific DNA sequences and thereby promote site-specific homologous recombination and targeted manipulation of genomic sequences.

As used herein the term "TAL-effector endonuclease" refers to artificial restriction enzymes generated by fusing a DNA recognition domain deriving from TALE proteins of ' Xanthomonas to a catalytic domain of a nuclease, as described by Voytas and Bogdanove in WO 2011/072246. TAL-effector endonucleases are named TALEN™ by the applicant (Cellectis, 8 rue de la Croix Jarry, 75013 PARIS).

In some advantageous embodiments, the RNP-complex used in the methods of the present disclosure comprises a nucleic acid comprising a crRNA and a tracrRNA, or a chimeric cr/tracrRNA hybrid, wherein the crRNA and tracrRNA, or the cr/tracrRNA hybrid, is targeted to a sequence that is endogenous to the cell; and a Cas9 endonuclease molecule that induces a double strand break at or near the sequence to which the crRNA and tracrRNA sequence is targeted, or at or near the sequence to which the cr/tracrRNA hybrid is targeted.

In one aspect, the present disclosure pertains to method for targeted genetic modification of a plant genome without inserting exogenous genetic material. The method can include (i) providing a plant cell that contains an endogenous gene to be modified, (ii) providing a genome-modifying formulation comprising a pre-assembled ribonucleoprotein (RNP)-complex comprising a nucleic acid modifying protein and a ribonucleic acid, (iii) delivering said RNP-complex into the cell by using physical approaches including mechanical approaches to get past the cell wall, and (iv) inducing one or more single or double stranded DNA breaks (DSB) in the genome, to produce a plant cell or cells having a detectable targeted genomic modification without the presence of any exogenous genetic material in the plant genome. The DSB can be repaired by non-homologous end joining (NHEJ).

The methods and compositions of the present disclosure may be applied to an intact differentiated cell like a plant cell, an animal cell, a human cell and a microbial cell. In particular, the cell may be comprised in a tissue and/or an organism. In some advantageous embodiments, the plant species used in the methods provided herein belong to the Solanaceae family and is *N. benthamiana or N. tabacum,* although in a further aspect the plant species may be any monocot or dicot plant, such as (without limitation) Arabidopsis thaliana; field crops (e.g., alfalfa, barley, bean, corn, cotton, flax, pea, rape, rice, rye, safflower, sorghum, soybean, sunflower, tobacco, and wheat); vegetable crops (e.g., asparagus, beet, broccoli, cabbage, carrot, cauliflower, celery, cucumber, eggplant, lettuce, onion, pepper, potato, pumpkin, radish, spinach, squash, taro, tomato, and zucchini); fruit and nut crops (e.g., almond, apple, apricot, banana, blackberry, blueberry, cacao, cherry, coconut, cranberry, date, fajoa, filbert, grape, grapefruit, guava, kiwi, lemon, lime, mango, melon, nectarine, orange, papaya, passion fruit, peach, peanut, pear, pineapple, pistachio, plum, raspberry, strawberry, tangerine, walnut, and watermelon); and ornamentals (e.g., alder, ash, aspen, azalea, birch, boxwood, camellia, carnation, chrysanthemum, elm, fir, ivy, jasmine, juniper, oak, palm, poplar, pine, redwood, rhododendron, rose, and rubber).

In some advantageous embodiments, the intact differentiated plant cell is isolated from plant leaves, flowers, seeds, roots or cotyledons before providing the pre-assembled ribonucleoprotein (RNP)-complex.

In some advantageous embodiments, the present disclosure pertains to methods for altering the genome of an intact differentiated cell(s) without inserting exogenous genetic material into the genome comprising:
(i) providing an intact differentiated cell that comprises an endogenous gene to be modified;
(ii) providing a genome-modifying formulation comprising a pre-assembled ribonucleoprotein (RNP)-complex comprising a nucleic acid modifying protein and a ribonucleic acid;
(iii) delivering said RNP-complex into the cell by using a physical delivery method;
(iv) inducing one or more single or double stranded DNA breaks in the cell genome to produce a cell having a detectable targeted genomic modification without the presence of any exogenous genetic material in the cell genome. Furthermore, the pre-assembled ribonucleoprotein (RNP)-complex may be co-delivered with one or more components, including but not limited to dNTP, ddNTP, non-natural base analoga, di-nucleotides, trinucleotides, oligonucleotides, enzyme inhibitors, sugars, amino acids, proteins, antibodies, transcription factors and other DNA-binding proteins, ribonucleic acids mRNA, siRNA, miRNA.

The RNP complexes can be delivered into the cell like the plant cell by using physical delivery methods/approaches including e.g. mechanical methods to get past the cell wall and / or the cell membrane. In some advantageous embodiments, the pre-assembled RNP-complex is coated on a particle. For example, the RNP-complexes can be coated onto micro- or nanoparticles made of materials including but not limited to gold, tungsten, aluminum, nickel, mesoporous silicate, silver, quantum dots etc. and be delivered by e.g. particle bombardment. Alternatively, the RNP-complex can be coated onto magnetic particles and be delivered applying a magnetic field. In particular, the pre-assembled RNP-complex is coated on the particle by air-drying.

The RNP-complex, in solution or as a freeze-dried powder, can be delivered into plant cells by surface abrasion of the plant tissue aided by an abrasive including but not limited to Diatomaceous earth powder (Celite^{®}), silicon carbide powder, sandpaper, silicon carbide whiskers, etc. The abrasive substance and the RNP-complex are deposited on the leaf tissue (either simultaneously, sequentially or after being pre-mixed) and then a gentle rubbing of the tissue surface is performed with the aid of, for example, a finger, a cotton pad, a piece of cloth etc. Alternatively, the plant tissue, the abrasive and the RNP-complex can be introduced in a container in either the presence or absence of a solution, and a shear force including but not limited to stirring, shaking, or vibrating is applied. The RNP-complexes can also be pre-coated onto the abrasive and then applied to the plant tissue by rubbing, friction, attrition etc.

The RNP-complexes can also be linked to and / or encapsulated in viruses, virus-like particles or other biopolymers such as starch, cellulose, chitin, chitosan and / or be exposed on the surface of such materials as free components, fusion proteins or conjugates, and be introduced into the plant cell by methods such as bombardment or surface abrasion.

In addition, the composition comprising the RNP-complexes can also contain enzymatic functions such as cellulases, pectinases, lipases, collagenases etc. and / or specific ligands as for example to facilitate attachment to cell walls, cell membranes, extracellular receptors.

The RNP-complexes and / or the particulate components can further comprise a fluorescent dye and / or light absorbing structure and / or component with a refractive index match to enable the use of optical tweezers (originally called "single-beam gradient force trap").

Local heating generated by a pulse of radiation including but not limited to LASER, infra-red, NMR etc., can also be used to locally perforate the cell wall and plasma membrane enabling the uptake of the RNP-complexes from the surrounding medium, either passively or by applying a second physical treatment including but not limited to vacuum, electrophoresis, spraying, etc.

Sonoporation induced by e.g. ultrasonic frequencies, echo waves etc., can also be used to modify the permeability of the cell wall and / or plasma membrane to allow the introduction of the RNP-complexes into the cell.

In some advantageous embodiments, the delivery method is abrasion and/or perforation and/or said delivery method is a biolistic particle delivery method. In particular, the biolistic particle delivery method comprises the use of a mixture of a plurality of particles and at least one isolated RNP-complex. The RNP complexes are generated by preferably mixing equimolar amounts of Cas9 protein and RNA in the form of either crRNA and tracrRNA, or cr/tracrRNA hybrid in solution in a tube and incubating the mixture at room temperature for 10-20 min. A molar excess of RNA components can also be used. The solution containing one or multiple RNP complexes is mixed with the gold particles and kept on ice for 5 to 20 min, in particular for about 10 min, then the mixture is deposited onto the macrocarrier and allowed to air dry at room temperature.

In some advantageous embodiments, the intact cell(s) is obtained from a non-sterile environment and/or provided under a non-sterile environment, e.g. a plant that is grown in a green house, a growth chamber, a field, a habitat, or a natural environment.

In some advantageous embodiments the intact cell(s) are further cultivated in a non-sterile environment after the pre-assembled ribonucleoprotein (RNP)-complex has been delivered to said intact cells(s). Such non-sterile environments may e.g. comprise a laboratory, a room, a green house, a growth chamber, a growth cabinet, a field, a garden, or a natural environment.

In some advantageous embodiments, the intact differentiated cell(s) are exposed to stress condition before and/or while delivering the pre-assembled ribonucleoprotein (RNP)-complex, including but not limited to UV-stress, heat-stress, cold-stress, draught, reactive oxygen-species or chemicals.

In further embodiments, the genome-modifying formulation, in particular the (RNP)-complex does not comprise DNA. Direct delivery of RNP complexes for genome editing is highly desirable because it does not involve DNA, precluding the possibility of the resulting plant to be transgenic.

In particular, the genome-modifying formulation, in particular the (RNP)-complex does not comprise a marker gene. A marker is a detectable genetic trait or segment of DNA that can be identified and tracked. A marker gene typically serves as a flag for another gene, sometimes called the target gene. A marker gene is typically used with a target gene being used to transform target cells. Target cells that heritably receive the target gene can be identified by selecting for cells that also express the marker gene. The marker gene is near enough to the target gene so that the two genes (the marker gene and the target gene) are genetically linked and are usually inherited together.

In some advantageous embodiments, the genome-modifying formulation comprising a plurality of different pre-assembled ribonucleoprotein (RNP)-complex, in particular the ribonucleoprotein (RNP)-complex differ in the nucleic acid sequence of the ribonucleic acid comprised in the complex. Therefore, the genome-modifying formulation comprises a plurality of pre-assembled ribonucleoprotein (RNP)-complexes, in particular with different ribonucleoprotein (RNP)-complexes. In some embodiments, the different ribonucleoprotein (RNP)-complexes can (A) be mixed and coated onto the same particle or they can (B) be coated separately onto the particles and then the coated particles could be mixed. Scenario (A) is more useful for the "targeted" approach and standard applications where an individual particle (=carrier) delivers a plurality of RNP-complexes to a single cell.

In some advantageous embodiments, the intact differentiated cell is exposed to the pre-assembled ribonucleoprotein (RNP)-complex, in particular in the presence of a carrier material, wherein after delivering said RNP-complex into the cell, the genome of the cell is altered/modified, and wherein after the modification/alteration the cells are further cultivated to derive a cell, cell line, tissue or organism with a modified (altered) genome without the presence of a marker gene.

### Methods and Examples

In the following examples, materials and methods of the present disclosure are provided. It should be understood that these examples are for illustrative purpose only and are not to be construed as limiting this disclosure in any manner. All publications, patents, and patent applications cited herein are hereby incorporated by reference in their entirety for all purposes.

### Example 0a: Delivery of DsRed protein by particle bombardment into intact leaf-tissue of N. benthamiana.

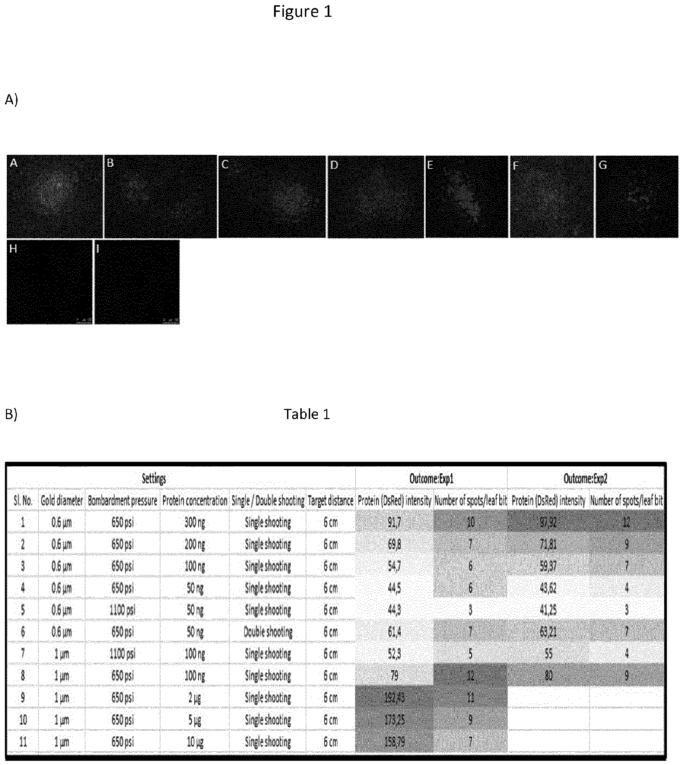

To test the delivery of proteins into plant cell with intact cell walls we selected the method of particle bombardment and recombinant DsRed protein (Discosoma sp. red fluorescent protein gene; R2G mutant; Sack et al., 2015) for the initial experiments. Different amounts of DsRed protein (50 ng, 100 ng, 200 ng, 300 ng, 2 µg, 5 µg and 10 µg) were used in these tests. The concentration of the DsRed protein stock solution was 50 µg/µL and it was diluted with Milli-Q water to the desired concentration for the bombardment. The microcarrier, i.e. gold particles (Bio-Rad, USA; catalog number - 1652263), were resuspended in Milli-Qwater at a concentration of 30 µg/µL. Ten microliter volume of the diluted DsRed protein solution was added to 2 µL of the gold particle suspension, mixed gently and incubated on ice for 10 minutes. Immediately after that, the entire 12 µL of the suspension was pipetted onto the macrocarrier (Bio-Rad, USA; catalog number - 1652335) and allowed to air dry on a horizontal flow bench at room temperature (22°C) for ca. 2 h. Gold particles without DsRed were used as a negative control. Young leaves from 4-6 weeks old *N.benthamiana,* obtained from the glass house (growth conditions are e.g. described e.g. in Sack et al., 2015; Buyel and Fischer, 2012) were used for bombardment using a PDS1000 Biolistic particle accelerator (Bio-Rad, USA). The young leaves were detached from the plant, the mid-rib was removed and the remaining central leaf area was cut in small bits approximately 10 mm x 5 mm in size using a scalpel. Two hours prior to bombardment the leaf bits were placed in a petri dish with solidified MSO medium (4.3 g/L MS salts + 125 mM Sorbitol + 125 mM mannitol, 7% agar, pH 5.8). The leaf bits were arranged in a circle of around 2.5 cm diameter at the center of the petri dish. Two different helium gas pressures (650 psi and 1100 psi) and gold particle size (diameter of 0.6 µm and 1.0 µm (Bio-Rad, USA)) were used following the instructions described in the standard Bio-Rad Biolistic^{®}PDS-1000/He Particle Delivery System protocol. The target distance was 6 cm. After bombardment, the leaf bits were placed on a wet filter paper in a petri dish and incubated in the light at 20 - 23°C. The effects of single and multiple bombardments were also investigated. In the case of double bombardment, the petri dish containing the leaf pieces was turned 90° after the first shot, and then bombarded again with the same settings. Fluorescence microscope images (Leica DMI 6000B, excitation at 563 nm and emission at 581 nm) were taken 4 h after bombardment keeping the microscope settings constant for all samples. DsRed fluorescence was not distributed evenly throughout the whole bombarded leaf area, but rather found concentrated in only a few isolated spots. The number of fluorescent spots was counted by eye, while fluorescence intensity was calculated using ImageJ. The experiments were performed twice, and the representative images and the results are summarized in Figure 1 and Table 1.

The data demonstrated a dose-dependency regarding both the number of DsRed fluorescent spots and the observed fluorescence intensity. The results clearly showed that DsRed fluorescence was confined to individual areas within the leaf tissue and that there were large sections that did not exhibit any significant DsRed fluorescence.

### Example 0b: Delivery of DsRed protein by particle bombardment into dedifferentiated plant suspension cells.

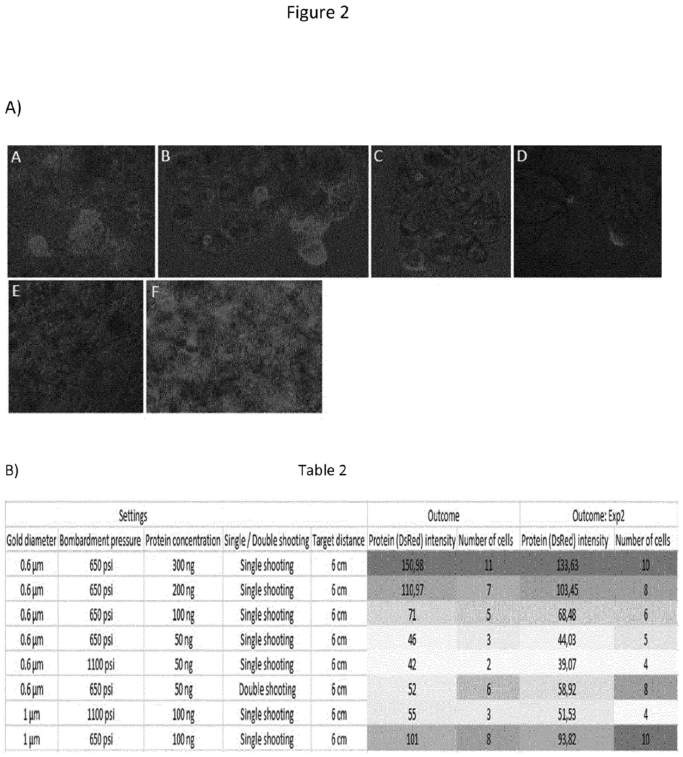

To deliver proteins into *Nicotiana tabacum* cv. BY2 suspension cells we used the same experimental conditions as described above. 50 mL of fresh culture medium (4.3 g/L MS, 30 g/L sucrose, 0.2 g/L KH₂PO₄, 0.2 mg/L 2,4 D, 1.0 mg/L Thiamin, 0.1 g/L Myoinositol, pH 5.8) were inoculated with 3 mL from a 7-day old suspension culture and incubated for 2 days as described in the reference (Nagata et al., 1992). After two days, the cell density was adjusted to packed cell volume (PCV) of 12% (v/v) using culture medium. The suspension was casted into a Büchner funnel with filter paper (Macherey-Nagel, Germany, 0.16 mm thickness and 5.5 cm diameter), and the liquid was removed by vacuum filtration. The volume of the BY2 culture passed over the filter paper was 10 mL per filter. The filter paper with the retained cells was placed on MSO medium (see Example 0a) for 2 h prior to bombardment. The bombardment parameters and the particle gun were the same as in Example 0a. After bombardment, the filter paper with the cells was transferred onto a wet filter paper in a petri dish and incubated for 4 h in the light. Then microscopic (Leica DMI 6000B) images were taken under white light and fluorescent light. The number of fluorescent cells was counted by eye, while fluorescence intensity was calculated using ImageJ. The experiments were performed twice, and representative images and the results are summarized in Figure 2 and Table 2, respectively. The data demonstrated a clear dose dependency for the number and intensity of the spots. The DsRed fluorescence was seen only as spots i.e. localized to individual cells or cell clusters.

### Example 0c: Agroinfiltration of SSA constructs in N.benthamiana

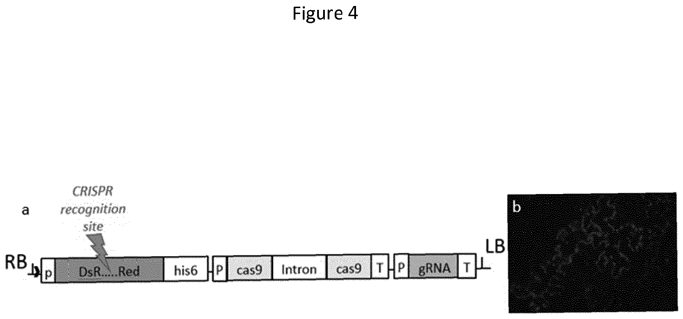

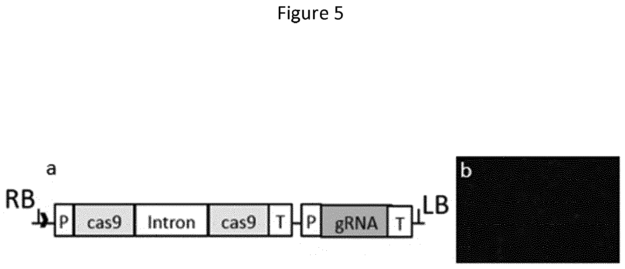

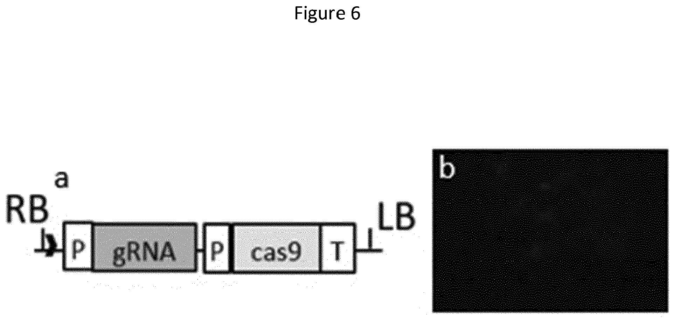

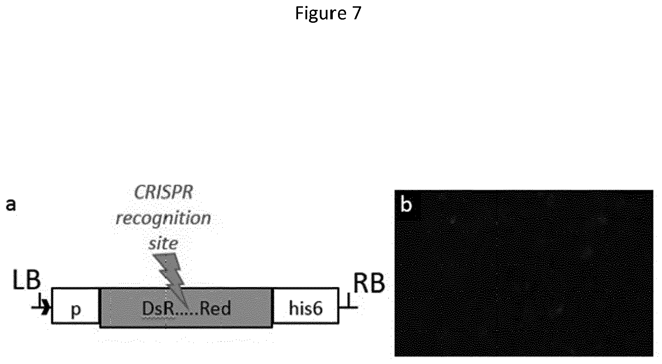

The binary vector pUlli1250 (Figure 3) containing the sequence for a DsRed-based single strand annealing (SSA) reporter was created. Briefly, the plasmid encodes two truncated inactive parts of the DsRed gene with overlapping directly repeated sequences flanking a CRISPR/Cas9 target sequence named "S3", which is a validated target in the human AAVS1 locus taken from Cho et al., 2014 (doi:10.1101/gr.162339.113). Following a double strand break at the target site a functional DsRed gene is reconstituted, and thus fluorescence is generated. Also, we generated a binary plasmid carrying the SSA reporter together with the Cas9 and gRNA genes (pUlli1244, Figure 4a) as a positive control, as well as negative control constructs carrying only the Cas9 (pUlli1232, Figure 5a) or sgRNA gene (pUlli541, Figure 6a). *N.benthamiana* was cultivated as described previously (Sack et al., 2015; Buyel and Fischer, 2012) and 4-6 weeks old plants were used for the agro-infiltration experiments. The recombinant Agrobacteria (GV3101, pMP90RK) carrying the respective binary plasmids pUlli1224, pUlli1232 or pUlli541 were grown in YEB medium supplemented with 2 mM MgSO4, 50 µg/mL Carbenicillin, 25 µg/mL Rifampicin and 25 µg/mL kanamycin and cultivated as described (Boes et al., 2016). The freshly grown bacterial cultures were diluted to an OD600=2 using water. The diluted cultures were then mixed in equal parts with 2x infiltration medium (10% (w/v) sucrose, 0.4% (w/v) glucose, 0.01% (w/v) Ferty2 Mega (Planta Düngemittel GmbH, pH 5.6) and acetosyringone (200 µM final concentration). The bacterial suspensions were incubated in the dark at room temperature for 1h and then infiltrated into *N.benthamiana* plants by manual injection with a needleless syringe. The infiltrated plants were incubated for 5 days at room temperature in a growth chamber with 16h light. Then small leaf bits were collected and analyzed using a fluorescence microscope. The constructs were infiltrated on different leaves of the same plant and the experiment repeated thrice. The data demonstrated that neither the expression of the CRISPR components alone (Figure 5b: pUlli1232; Figure 6b: pUlli541) nor of the SSA construct alone (Figure 7b: pUlli1250) resulted in detectable DsRed fluorescence. In contrast, when the leaves were infiltrated with Agrobacteria harboring the plasmid pUlli1244 containing all elements together, high DsRed expression throughout the leaf was observed (Figure 4b). These results show that the plasmid pUlli1250 encodes a suitable SSA reporter gene that gives a readily visible readout of Cas9 activity.

### Example 1: Targeting the single strand annealing (SSA) construct delivering a ribo-nucleo-protein (RNP) complex into N. benthamiana leaf tissue by particle bombardment

The binary vector pUlli1250 containing the sequence for the DsRed-based single strand annealing (SSA) reporter described in example 0c was used. Middle-sized leaves from 4-6 weeks old *N.benthamiana* plants grown in the greenhouse as described previously (Sack et al., 2016; Buyel and Fischer, 2012) were used. Leaves were infiltrated with Agrobacteria carrying the construct pUlli1250 as described above using a needleless syringe (Boes et al., 2016). After incubation for one day in growth chamber at room temperature with 16h light, the agroinfiltrated leaves were detached from the plant, the mid-rib was removed, the remaining central leaf area was cut into small bits approximately 10 mmx 5 mm, and these were placed in a petri dish with solid MSO medium for 2 h as described in example 0a. The RNP complexes were generated using equimolar amounts of Cas9 protein (Alt-RTM S.p. Cas9 nuclease 3NLS; Integrated DNA Technologies-IDT), crRNA (AltRTM CRISPR crRNA; IDT) and tracrRNA (AltRTM CRISPR tracrRNA; IDT) following the instructions described in the standard IDT Alt-R™CRISPR-Cas9 system user guide.

Different amounts of cas9 per shooting were used (here referred to as the amount of Cas9 in weight, and as moles in parenthesis), namely: 1 µg (0.0091 nmol), 10 µg (0.091 nmol), 25 µg (0.228 nmol) and 50 µg (0.458 nmol).

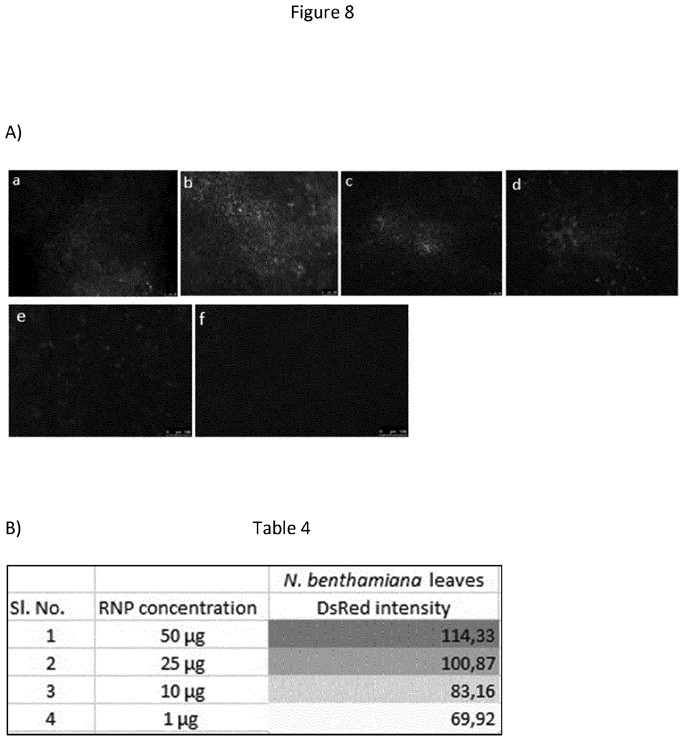

One crRNA targeting the CRISPR/Cas9 recognition site in plasmid pUlli1250 (GGGAGGGAGAGCTTGGCAGGGGG) was used together with the tracrRNA (IDT). The RNP suspension (10 µL) was mixed with 2 µL of gold, mixed thoroughly and kept on ice for 10 min. Immediately after that the entire 12 µL of the suspension was pipetted onto a macrocarrier (Bio-Rad, USA; catalog number - 1652335) and allowed to air dry on a horizontal flow bench for 4-6 h at room temperature (∼22°C). The parameters used for bombardment were 1 µm gold, 6 cm target distance and 650 psi pressure. After bombardment, the leaf bits were placed on a wet filter paper in a closed petri dish and incubated in a controlled growth chamber at 20-22°C, 16 h photoperiod (7000 lux). The samples were analyzed by fluorescence microscopy 3 days after bombardment. The experiment was repeated twice. A high DsRed fluorescence was observed by fluorescence microscopy (Figure 8) and the DsRed intensities were quantified by Image J (Table 4).

This example shows that an RNP complex delivered by particle bombardment to intact cells of *N. benthamiana* leaves results in efficient DNA cleavage as demonstrated by the high DsRed fluorescence arising from the single strand annealing (SSA) reporter construct encoded on the binary vector pUlli1250.

Surprisingly, in contrast to example 0a, the DsRed fluorescence was observed in a high number of the cells, showing that the method according to this invention is highly efficient.

### Example 2: Targeting the single strand annealing (SSA) construct by delivering a RNP complex into N. tabacum BY2 suspension cells by particle bombardment.

BY2 cells were cultivated and collected as described in example 0b. After removing the liquid media by vacuum filtration, the BY2 cells were transformed with a suspension of recombinant Agrobacteria (OD₆₀₀ of 1.0) carrying the binary T-DNA vector pUlli1250 using the method described in WO 2013113504 A1. The filter with the cells was kept in an incubator at 26°C with 80 % humidity for 1 day. Then the filter with the cells was transferred onto MSO medium for 2 h. The RNP complex and gold particles were prepared as described in example 1 and the parameters for bombardment were also the same as in example 1. After bombardment, the filter paper with the cells was placed on a wet filter paper in a petri dish and incubated (26°C with 80 % humidity) for three days. Then the cells were analyzed by fluorescence microscopy.

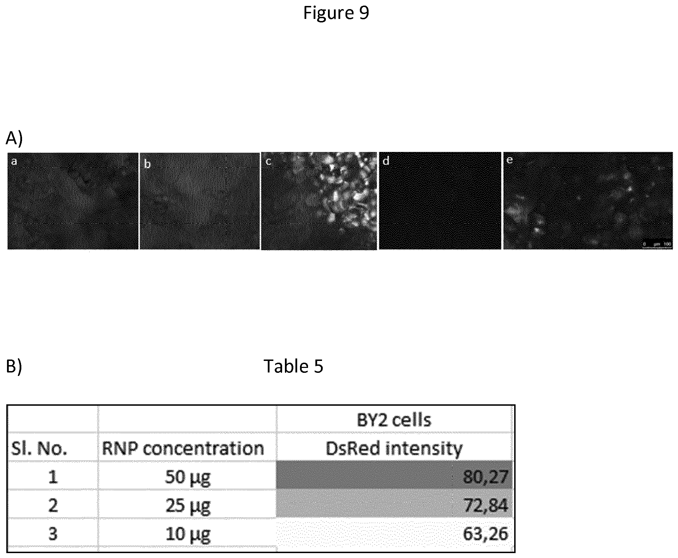

A high DsRed intensity was observed (Figure 9) and the intensities were quantified by Image J (Table 5). This example shows that bombardment with a RNP complex results in efficient DNA cleavage in suspension cells of *Nicotiana tabacum* cv. BY2 that have intact cell walls.

### Example 3: Targeting the endogenous tobacco pds gene by particle bombardment of RNP.

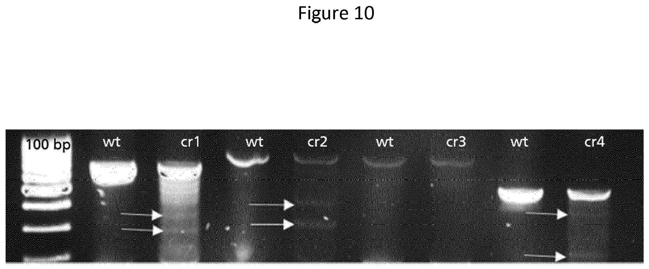

We selected 4 crRNA targeting the tobacco pds gene; the sequences are given in Table 6. The crRNA 1, 3 and 4 were obtained from Gao et al. (2015), and crRNA 2 was newly designed. All the crRNAs were tested individually using in vitro grown (Pospisilova J et al., 1998) 4 months old *N. tabacum* leaves. The young leaves were cut into pieces of approximately 10 mm x 5 mm and placed on MSO medium for 2 h prior to bombardment. The parameters for the particle bombardment were the same as in example 1. The RNP complex was prepared following the conditions described in the standard IDT Alt-R™CRISPR-Cas9 system user guide. The RNP suspension was mixed with 2 µL of gold suspension (30 µg/mL, catalog number - 1652263), mixed thoroughly and kept on ice for 10 min. Immediately after that the suspension (12 µL final volume) was pipetted onto a macrocarrier and allowed to air dry on a horizontal flow bench for 4-6 h at 22°C. After bombardment, the leaves were placed on a wet filter paper under light and 24 h later genomic DNA was extracted from the leaf bits using the Nucleospin Plant II kit (Macherey Nagel, Germany). PCR amplifications of the target region were done using the primers listed in Table 7. For crRNA 2, 3 and 4 the thermocycler was set for one cycle 98°C for 30 sec, 30 cycles of 98°C for 10 s, 57°C for 30 s, 72°C for 30 sec and 72°C for 7 min and held at 4°C . For crRNA 1 the thermocycler was set for one cycle 98°C for 30 sec, 30 cycles of 98°C for 10 s, 56°C for 30 s, 72°C for 30 sec and 72°C for 7 min and held at 4°C. The rate of cas9 activity was measured by T7 endonuclease I digestion of hybridized PCR products. PCR DNA (200 - 600 ng) in 1x NEB Buffer 2 was denatured and hybridized in a thermocycler (Bio-Rad, USA) under the following conditions: 95°C for 10 min, 95-85°C at -2°C s-1, 85- 15°C at -0.2°C s-1 and then cooled down and held at 4°C. Ten units of T7 EI (NEB, M0302L) were added to the sample followed by incubation at 37°C for 1 h. A PCR fragment derived from wild type *N. tabacum* was used as a control. The samples were then immediately separated on a 2% agarose gel containing ethidium bromide (Figure 10).

This example demonstrates efficient and rapid genome editing of an endogenous gene in *Nicotiana tabacum.*

**Table 6: crRNA sequences, the PAM region is marked in red font**

| **crRNA** | **Target region sequence 5' → 3'** |
|---|---|
| crRNA 1 | GCCGTTAATTTGAGAGTCCAAGG |
| crRNA 2 | TTTGGAATATCAGGTTTGGGTGG |
| crRNA 3 | GAGGCAAGAGATGTCCTAGGTGG |
| crRNA 4 | GCTGCATGGAAAGATGATGATGG |

**Table 7: Primer sequences used for amplifying the target regions**

| **crRNA** | **Primer sequence 5' → 3'** |
|---|---|
| crRNA 1 F | CTTGATTTTGTGGGTGAAGGA |
| crRNA 1 R | ATGGTTTAGTTGGGCGTGAG |
| crRNA 2 & 3 F | CTAAGGCTTGCAATCTGTGAG |
| crRNA 2 & 3 R | CCATATCTTTTACCTAATATGCTGTAC |
| crRNA 4 F | GTACAGCATATTAGGTAAAAGATATGG |
| crRNA 4 R | GCAAGGCAGAATACAGATCG |

### Example 4: Targeting different regions of the endogenous N. tabacum pds gene by simultaneous particle bombardment with multiple RNPs.

Because the ease, speed and efficiency of the genome editing according to this invention was so unexpectedly high, we proceeded to simultaneously target two regions in the *pds* gene of *N. tabacum.* For this, combinations of crRNAs were used i.e. crRNA 2 and 4, crRNA 2 and 3 and crRNA 3 and 4 were chosen. The experiment was conducted as described in example 3, with the exception that a mixture of RNP complexes (equimolar amount of both the crRNAs) was prepared. This was done by mixing 25 µg of each gRNA and 25 µg of cas9 for each gRNA.

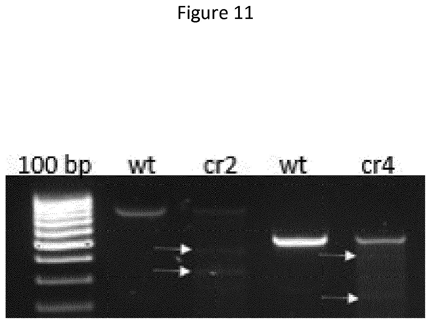

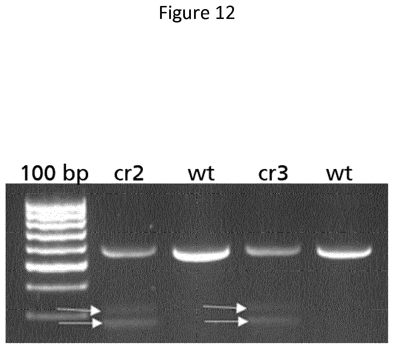

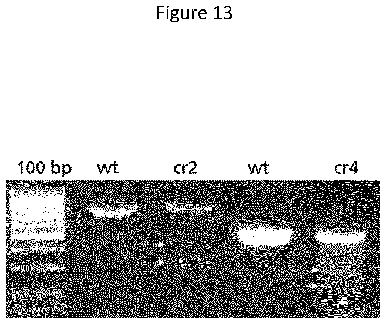

Immediately after that, the suspension comprising the RNP mixture (18 µL final volume) was pipetted onto a macrocarrier and allowed to air dry on a horizontal flow bench for 4-6 h at 22°C. After bombardment, the leaves were placed on a wet filter paper under light at 20 -23°C and 24 h later genomic DNA was extracted using the Nucleospin Plant II kit (Macherey Nagel, Germany). PCR amplification of the target sites and T7 EI assay (Figure 11, 12 and 13) were done as per example 3. The primer details and PCR conditions are explained in example 3.

This example clearly demonstrates that two target regions were efficiently and simultaneously modified.

### Example 5: Regeneration of plants from N. tabacum leaves grown under sterile conditions bombarded with a mixture of RNPs without using a selection marker or a scorable marker.

After bombardment with multiple crRNAs (crRNA 2 and 4, 2 and 3 and 3 and 4) as described in example 4, the leaf pieces were kept for one day at 20 -23°C and 16h light. Then the leaves were transferred to MS medium (4.4 g/L MS-salts with vitamins (Duchefa M0222), 20 g/L Sucrose, 0.6 mg/L Thiamine-HCl, 7 g/L agar, pH 5.8) with hormones (1 mg/L 6-BAP, 0.1 mg/L NAA) and kept in the dark for 2 days at 20 - 23°C. Then the leaves were transferred to light under controlled conditions (16 h Photoperiod, 20-23°C), transferring them onto fresh plates every 2 weeks until shoots appeared (Pospisilova J et al., 1998). Shoots were transferred onto MS medium, incubated at 20-25°C with a 16 h photoperiod (7000 lux), until roots developed and finally transferred into soil and grown in the greenhouse in ED73 standard soil (Patzer) with 0-30 % (v/v) sand with 16 h illumination of 10000 lux (plus the sun light) and 70-90 % humidity. Tobacco plants were generated without applying any selection.

This example demonstrates that the efficiency of genome editing using the method according to this invention is so unexpectedly high that it enables the generation of genome edited plants without the use of a selection marker (such as an antibiotic or herbicide resistance gene) or scorable marker (such as a fluorescent protein, enzyme or genes generating pigments).

This is very surprising because it is generally difficult to generate transgenic plants even harboring a highly expressed transgene without using a selectable or scorable marker gene. When foreign DNA is delivered to the plant cell it is introduced into the plant genome and then replicated during mitosis. In contrast, the RNP complex delivered to the plant cell cannot be replicated in any way and is subject to degradation in the plant cell. Moreover, during cell division the RNP is further diluted and thus eventually will not be present in the plant cells, tissue or whole plant any more.

Notably, the method according to this invention also enables the generation of genome modified plants without using recombinant DNA and this is a tremendous advantage for subsequent industrial use, in particular for growing genome edited plants in the field or other non-contained environments. Plants generated using the method of this invention are not transgenic plants.

Finally, the results demonstrate that the method according to this invention facilitates the simultaneous modification of two target regions.

### Example 6: Delivery by particle bombardment of multiple RNPs to N. tabacum plants grown under non-sterile conditions and regeneration of genome modified plants.

The crRNAs 2 and 4 were multiplexed in glass house grown *N.tabacum* leaves (Sack et al., 2015; Buyel et al., 2012) through particle bombardment. The conditions for making the RNP complex and the parameters for bombardment were the same as in example 4. After bombardment, the leaves were placed on a wet filter paper under light at 20 - 23°C and 24 h later surface sterilization (70% ethanol momentarily, soaked in 5% sodium hypochlorite for 10 min, and rinsed 3 times with sterile water) was done and the leaf pieces were further processed for regeneration as described in Example 5.

This examples shows that the method according to this invention enables the use of organisms, tissues and/or cells grown under non-sterile conditions or collected from non-sterile environments.

### Example 7: Delivery of RNP by particle bombardment into tomato leaves

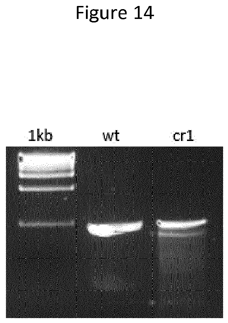

We selected a crRNA targeting the tomato pds gene (Pan et al., 2016), which sequence is listed in **table 8.** Tomato (MicroTom) plants were grown aseptically in tissue culture as described in (McCormick et al., 1986, Fillati et al., 1987). The young leaves were cut into pieces of approximately 10 mm x 5 mm and placed on MSO medium 2 h prior to bombardment. The parameters for the particle bombardment were the same as in example 1. The RNP complex was prepared following the conditions described in the standard IDT Alt-R™CRISPR-Cas9 system user guide. The RNP suspension was mixed with 2 µL of gold suspension (30 µg/mL, catalog number - 1652263), mixed thoroughly and kept on ice for 10 min. Immediately after that, the suspension was pipetted onto a macrocarrier (12 µL final volume) and allowed to air dry on a horizontal flow bench for 4-6 h at 22°C. After bombardment, the leaves were placed on a wet filter paper under light at 20 -23°C and 24 h later genomic DNA was extracted from the leaves using the Nucleospin Plant II kit (Macherey Nagel, Germany). PCR amplification of the target region was done using the primers listed in **table 9.** The thermocycler was set for one cycle 95°C for 4 min, 30 cycles of 95°C for 30 s, 57°C for 30 s, 72°C for 1 min and 72°C for 7 min and held at 4°C. The rate of cas9 activity was measured by T7 endonuclease I digestion (**Figure 14**) as per example 3.

**Table 8: crRNA sequences, PAM region is marked in red font**

| crRNA | Target region sequence |
|---|---|
| crRNA | GGACTCTTGCCAGCAATGCTTGG |

**Table 9: Primer sequences for target region amplification**

| crRNA | Primer sequence |
|---|---|
| crRNA F | ATGGTGAGCTAATCACGAGTAA |
| crRNA R | AAAGAGAGGCAGGTAGACAATC |

### Example 8: Delivery of RNP by particle bombardment into tomato cells (MicroTom) and regeneration of genome edited / modified plants.

The crRNA targeting the tomato *pds* gene (Table 8; Pan et al., 2016) was delivered into *in vitro* grown (Pospisilova J et al., 1998) 10 -12 days old MicroTom cotyledons by particle bombardment. The cotyledons were placed on MSO medium 2 h prior to bombardment. The parameters for the particle bombardment were the same as in example 1. The RNP complex was prepared following the conditions described in the standard IDT Alt-R™CRISPR-Cas9 system user guide. The RNP suspension (more details needed here) was mixed with 2 µL of gold suspension (30 µg/mL, catalog number - 1652263), mixed thoroughly and kept on ice for 10 min. Immediately after that the suspension was pipetted onto a macrocarrier (12 µL final volume) and allowed to air dry on a horizontal flow bench for 4-6 h at room temperature (22°C). After bombardment, the leaves were placed on a wet filter paper under light at 20 -23°C and 24 h later the bombarded leaves were transferred to tomato regeneration medium (TRM: 4.4 g/L MS-salts with vitamins (Duchefa M0222), 20 g/L Sucrose, 1 ml/L Nitsch's vitamin, 2.25 g/L Gelrite, pH 6.0) with hormone (2 mg/mL Zeatin Riboside) and kept under dark for 2- 3 days at 20 - 23°C. After that, the leaves were transferred to controlled conditions (16 h Photoperiod, 20-23°C), transferring them onto fresh plates every 2 weeks until shoots appeared (Pospisilova J et al., 1998). Shoots were transferred onto TRM medium, incubated at 20-25°C with a 16 h photoperiod (7000 lux), until roots developed and finally transferred into soil and grown in the greenhouse in ED73 standard soil (Patzer) with 0-30 % (v/v) sand with 16 h illumination of 10000 lux (plus the sun light) and 70-90 % humidity.

This example shows that the method according to this invention is generally applicable to different plant species and not restricted to species of the *Nicotiana* family.

### Example 9a: Delivery of the DsRed protein into N. benthamiana leaves by abrasion with Celite-503.

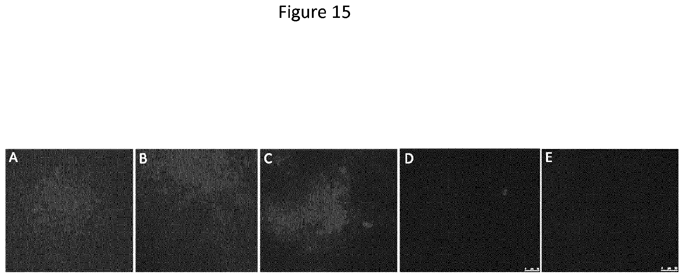

The DsRed protein was delivered into leaves of 4-6 weeks old *N.benthamiana* plants by abrasion with Celite-503. The plants were grown in the glass house as described previously (Sack et al., 2015; Buyel et al., 2012). The DsRed protein stock solution (50 µg/µL) was diluted with Milli-Qwater to the desired concentration (1 µg/µL, 0.5 µg/µL and 0.2 µg/µL). MilliQ water only was used as negative control. The dry Celite-503 powder was evenly dispersed onto the leaves from a 15 mL conical tube through 4 layers of cheesecloth. The abrasive was almost invisible on the leaf. Then 10 µL of either the DsRed protein solution or MilliQ water was placed on the leaf and spread by rubbing with 10 soft but firm strokes with a gloved finger. After that, the leaves were gently washed with water to remove the excess of Celite-503 (Ascencio-Ibanez and Settlage, 2007) and cut it into small pieces approximately 10 mm x 5 mm size. The leaves were then placed on a wet filter paper and incubated under light at 20 -23°C. Fluorescence microscope images (Leica DMI 6000B) were taken 4 h after the treatment. Representative images are shown in Figure 15.

### Example 9b: Delivering the RNP into tobacco leaves by abrasion with Celite-503.

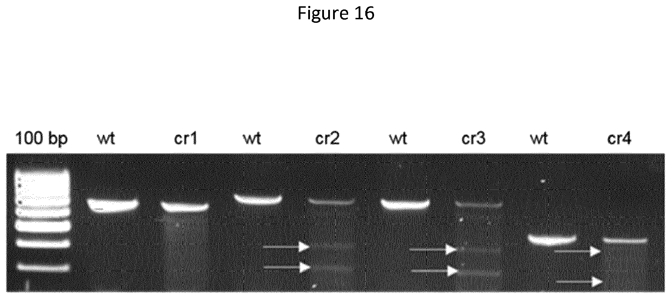

The crRNAs targeting the tobacco pds gene (crRNA 1, 3 and 4 were obtained from Gao et al (2014) and crRNA 2 was newly designed) described in example 3 were delivered to leaves of 4 months old plants of *N. tabacum* by abrasion with Celite-503. The plants were grown aseptically in tissue culture as described previously. The RNP complex was prepared following the conditions described in the standard IDT Alt-R™CRISPR-Cas9 system user guide. Ten µL of the RNP solution were added to 10 µL of cas9 buffer (20 mM HEPES, 150 mM KCL, pH 7.5), yielding a solution of 20 µL. A minimal amount of dry Celite-503 was evenly dispersed onto the leaves from a 15 mL conical tube through 4 layers of cheesecloth. The abrasive was almost invisible on the leaf. Then the 20 µL of the RNP solution was placed on the leaf and spread by rubbing with 10 soft but firm strokes with a gloved finger. After that, the leaves were gently washed with water to remove the excess of Celite-503 (Ascencio-Ibanez and Settlage, 2007) and cut it into small pieces approximately 10 mm x 5 mm size. The leaves were then placed on a wet filter paper and incubated under light at 20 -23°C. After 24 h genomic DNA was extracted from the leaf bits using the Nucleospin Plant II kit (Macherey Nagel, Germany). PCR amplifications of the target region was done using the same primers (**table 7**) and the same PCR conditions described in example 3. The rate of cas9 activity was measured by T7 endonuclease I digestion (Figure 16) of hybridized PCR products as per the example 3.

### Example 9c: Delivery of multiple RNPs into tobacco leaves by abrasion with Celite-503.

As in the example 4, crRNA 2 and 4 were used to simultaneously target two sites of the of *N. tabacum* pds gene using Celite-503 to deliver the RNP to intact leaves by abrasion.

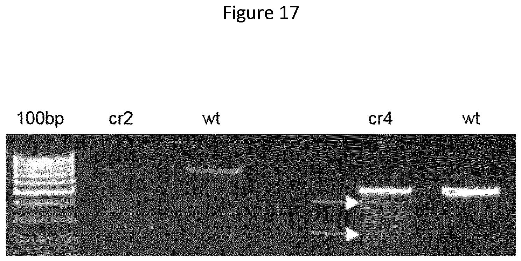

For multiplexing, *in vitro* grown (Pospisilova J et al., 1998) 3 - 4 months old *N. tabacum* leaves were used and the RNP complexes were introduced by abrasion with Celite-503. The RNP complexes were prepared following the conditions described in the standard IDT Alt-R™CRISPR-Cas9 system user guide. Abrasion with Celite-503 was done as per example 9a. After treatment, the leaf pieces were placed on a wet filter paper under light at 20 -23°C and 24 h later genomic DNA were extracted using the Nucleospin Plant II kit (Macherey Nagel, Germany). PCR amplifications of the target regions was done using the same primers and PCR conditions are described in example 3, **table 7.** The rate of cas9 activity was measured by T7 endonuclease I digestion (**Figure 17**) of hybridized PCR products as per example 3.

This example shows that the method according to this invention is not restricted to ballistic delivery methods such as particle bombardment but works equally well with other delivery methods such as surface abrasion with Celite-503.

### Example 9d: Delivery of RNP into tobacco leaves by abrasion with Celite-503 and regeneration of genome edited plants.

After abrasion with Celite-503 with a mixture of two RNPs targeting the tobacco pds gene (crRNA 2 and 4) as described in example 9b, the treated leaves were kept in the light at 20 -23°C for 24 h. After that, the leaf bits were transferred to plates with MS regeneration medium (MS Medium: 4.4 g/L MS-salts with vitamins (Duchefa M0222), 20 g/L Sucrose, 0.2 mg/L Thiamine-HCl, pH 5.8) with hormones (1 mg/L 6-BAP, 0.1 mg/L NAA) and kept in the dark for 2 days at 20 - 23°C. Then the leaves were transferred to controlled conditions (16 h Photoperiod, 20-23°C, transferring them onto fresh plates every 2 weeks) until shoots appeared (Pospisilova J et al., 1998). Shoots were transferred onto MS medium, incubated at 20-25°C with a 16 h photoperiod (7000 lux), until roots developed and finally transferred into soil and grown in the greenhouse in ED73 standard soil (Patzer) with 0-30 % (v/v) sand with 16 h illumination of 10000 lux (plus the sun light) and 70-90 % humidity.

The results also show that the method is efficient, fast and easy also for Celite-503 abrasion and enables simultaneous targeting of at least two regions in the plant genome using intact tissue as a starting material.

### Example 9E: Delivery of RNP by abrasion with Celite-503 into tomato cells (MicroTom).

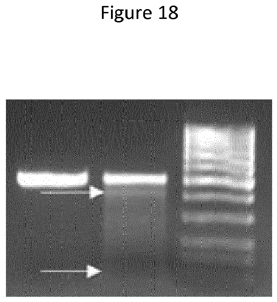

The crRNA for tomato pds gene (Table 7; Pan et al., 2016) was delivered into 3 - 4 months old glasshouse grown (Sack et al., 2015; Buyel et al., 2012) MicroTom leaves through abrasion with Celite-503. The RNP complex was prepared following the conditions described in the standard IDT Alt-R™CRISPR-Cas9 system user guide. Abrasion with Celite-503 was done as per the example 9a. After treatment, the leaves were placed on a wet filter paper under light at 20 -23°C and 24 h later genomic DNA was extracted using the Nucleospin Plant II kit (Macherey Nagel, Germany). PCR amplifications of the target region was done using the same primers and PCR conditions described in example 7, Table 8. The rate of cas9 activity was measured by T7 endonuclease I digestion (Figure 18) of hybridized PCR products as per the example 3.

### Example 9F: Delivery of RNP by abrasion with Celite-503 into tomato cells (MicroTom) and regeneration of genome edited / modified plants.

After abrasion with Celite-503 with a tomato RNP targeting the tomato pds gene as described in example 9E, the treated leaves were kept in the light at 20 -23°C for 24 and later it was transferred to tomato growth medium (TGM: 4.4 g/L MS-salts with vitamins (Duchefa M0222), 20 g/L Sucrose, 1 ml/L Nitsch's vitamin, 2.25 g/L Gelrite, pH 6.0) with hormone (2 mg/mL Zeatin Riboside) then kept in the dark for 2- 3 days at 20 - 23°C. After that, the leaves transferred to light under controlled conditions (16 h Photoperiod, 20-23°C, transferring them onto fresh plates every 2 weeks) until shoots appeared. Shoots were transferred onto TM medium, incubated at 20-25°C with a 16 h photoperiod (7000 lux), until roots developed and finally transferred into soil and grown in the greenhouse in ED73 standard soil (Patzer) with 0-30 % (v/v) sand with 16 h illumination of 10000 lux (plus the sun light) and 70-90 % humidity.

### Example 10a: Delivery of RNP into Petunia by particle bombardment and analysis of the mutation

We selected 2 crRNAs targeting the Petunia *pds* gene (Zhang et al., 2015), whose sequence is given in **table 10.** Petunia plants were grown in the glass house as described (Sack et al., 2015). The particle bombardment experiments were essentially performed as described above. The young leaves were cut into pieces of approximately 10 mm x 5 mm and placed on MSO medium 2 h prior to bombardment. The parameters for the particle bombardment were the same as in example 1a. The RNP complex was prepared following the conditions described in the standard IDT Alt-R™CRISPR-Cas9 system user guide. The RNP suspension was mixed with 2 µL of gold suspension (30 µg/mL, catalog number - 1652263), mixed thoroughly and kept on ice for 10 min. Immediately after that, the suspension was pipetted onto a macrocarrier (18 µL final volume) and allowed to air dry on a horizontal flow bench for 4-6 h at 22°C. After bombardment, the leaves were placed on a wet filter paper under light at 20 -23°C and 24 h later genomic DNA was extracted using the Nucleospin Plant II kit (Macherey Nagel, Germany). PCR amplification of the target regions was done using the primers listed in **table 10.** The thermocycler was set for one cycle 98°C for 30 sec, 30 cycles of 98°C for 10 s, 57°C for 30 s, 72°C for 30 sec and 72°C for 7 min and held at 4°C. The rate of cas9 activity was measured by T7 endonuclease I digestion as per example 3.

**Table 10: crRNA sequences, PAM region is marked in red font**

| **crRNA** | **Target region sequence in petunia pds gene** |
|---|---|
| crRNA 1 | GGTGTTCATTGCCATGTCAA |
| crRNA 2 | GGCATGCAAAGTCTCTCAGG |

**Table 11: Primer sequences for target region amplification**

| crRNA | Primer sequence |
|---|---|
| crRNA1 F | CCGAAGCTCTTCCTGCTCCAA |
| crRNA1 R | GGTTTCTCTGGGTTGAGGACATAC |
| crRNA2 F | GGCGAGATAGGATTCTAACCTGAAG |
| crRNA2 R | GGTACTCCGACTAACTTCTCCAACT |

### Example 10b: Delivery of RNP into Petunia by particle bombardment and regeneration of genome edited / modified plants

The crRNAs targeting the Petunia pds gene (Zhang et al., 2015), whose sequence is given in **table 10.** Petunia hybrida cv Mitchell (W115) plants were grown in the glass house as described (Sack et al., 2015, Buyel et al., 2012). The particle bombardment experiments were essentially performed as described above. The young leaves were cut into pieces of approximately 10 mm x 5 mm and placed on MSO medium 2 h prior to bombardment. The parameters for the particle bombardment were the same as in example 1a. The RNP complex was prepared following the conditions described in the standard IDT Alt-R™CRISPR-Cas9 system user guide. The RNP suspension was mixed with 2 µL of gold suspension (30 µg/mL, catalog number - 1652263), mixed thoroughly and kept on ice for 10 min. Immediately after that, the suspension was pipetted onto a macrocarrier (18 µL final volume) and allowed to air dry on a horizontal flow bench for 4-6 h at 22°C. After bombardment, the leaves were placed on a wet filter paper under light at 20 -23°C and 24 h later the bombarded leaves were transferred to MS-B5 medium [4.4 g/L MS salts with Gamborg B5 vitamins (Duchefa M0231), 30 g/L sucrose, 10 g/L glucose, 8 g/L agar] with hormones (1 mg/L folic acid, 2 mg/L 6-BAP, 0.1 mg/L NAA) and kept in the dark for 2- 3 days at 20 - 23°C. After that, the leaves were transferred to light under controlled conditions (16 h Photoperiod, 20-23°C), transferring them onto fresh plates every 2 weeks until shoots appeared (Pospisilova J et al., 1998). Shoots were transferred onto MS-B5 medium, incubated at 20-25°C with a 16 h photoperiod (7000 lux), until roots developed and finally transferred into soil and grown in the greenhouse in ED73 standard soil (Patzer) with 0-30 % (v/v) sand with 16 h illumination of 10000 lux (plus the sun light) and 70-90 % humidity.

This example shows that the method according to this invention is generally applicable to different plant species and not restricted to species of the genus *Nicotiana.*

### Example 11a: Delivery of RNP by abrasion with Celite-503 into Petunia and regeneration of genome edited / modified plants.

We selected a crRNAs targeting the Petunia pds gene (Table 10; Zhang et al., 2015) to be delivered into 2 months old glasshouse grown (Sack et al., 2015; Buyel et al., 2012) Petunia hybrida cv Mitchell (W115) leaves by abrasion with Celite-503. The RNP complex was prepared following the conditions described in the standard IDT Alt-R™CRISPR-Cas9 system user guide. Abrasion with Celite-503 was done as per the example 9a. After treatment, the leaves were placed on a wet filter paper under light at 20 -23°C and 24 h later genomic DNA was extracted using the Nucleospin Plant II kit (Macherey Nagel, Germany). PCR amplifications of the target region was done using the primers in example 10a, table 10 and PCR conditions are described in example 10a. The rate of cas9 activity was measured by T7 endonuclease I digestion of hybridized PCR products as per the example 3.

### Example 11b: Delivery of RNP by abrasion with Celite-503 into Petunia and regeneration of genome edited / modified plants.

The crRNAs targeting the Petunia *pds* gene (Table 10; Zhang et al., 2015) to be delivered into 2 months old glasshouse grown (Sack et al., 2015; Buyel et al., 2012) Petunia hybrida cv Mitchell (W115) leaves by abrasion with Celite-503. The RNP complex was prepared following the conditions described in the standard IDT Alt-R™CRISPR-Cas9 system user guide. Abrasion with Celite-503 was done as per the example 9a. After treatment, the leaves were placed on a wet filter paper under light at 20 -23°C and 24 h later the leaves were transferred to MS-B5 medium (20 g/L sucrose, 10 g/L glucose, 4.4 g/L MS, 8 g/L agar, pH 5.7-5.9) with hormones (1 mg/L folic acid, 2 mg/L 6-BAP and 0.1 mg/L NAA) then kept under dark for 2- 3 days at 20 - 23°C. After that, the leaves transferred to light under controlled conditions and processed for plant regeneration as described in Example 10b.

### References

Barbora Hana c kova and J. Snopek (1998) Acclimation of tobacco plantlets to ex vitro conditions as affected by application of abscisic acid. Journal of Experimental Botany, Vol. 49, No. 322, pp. 863-869.
Boes A, Holger Spiegel, Robin Kastilan, Susanne Bethke, Nadja Voepel, Ivana Chudobová, Judith M. Bolscher, Koen J. Dechering, Rolf Fendel, Johannes F. Buyel, Andreas Reimann, Stefan Schillberg and Rainer Fischer. (2016). Analysis of the dose-dependent stage-specific in vitro efficacy of a multi-stage malaria vaccine candidate cocktail. Malaria Journal. 15:279
Buyel JF, Fischer R. (2012). Predictive models for transient protein expression in tobacco (Nicotiana tabacum L.) can optimize process time, yield, and downstream costs. Biotechnol Bioeng. Oct;109(10):2575-88.
Fillati J J, Kiser J, Rose R and Comai L. (1987). Efficient transfer of a glyphosate tolerance gene into tomato using a binary Agrobacterium tumefaciens vector; Bio/Technology 5:726-731.
Gao J, Wang G, Ma S, Xie X, Wu X, Zhang X, Wu Y, Zhao P, Xia Q. (2014). CRISPR/Cas9-mediated targeted mutagenesis in Nicotiana tabacum. Plant Mol Biol. 87(1-2):99-110.
Jinek M, Chylinski K, Fonfara I, Hauer M, Doudna JA, Charpentier E. (2012) A programmable dual-RNA-guided DNA endonuclease in adaptive bacterial immunity. Science. 337, 816.
Kim J and Kim J-S. (2016) Bypassing GMO regulations with CRISPR gene editing. Nature Biotechnology 34, 1014-1015.
Liang Z, Chen K, Li T, Zhang Y, Wang Y, Zhao Q, Liu J, Zhang H, Liu C, Ran Y and Gao C. (2017). Efficient DNA-free genome editing of bread wheat using CRISPR/Cas9 ribonucleoprotein complexes. Nature Communications 8, Article number: 14261.doi:10.1038/ncomms14261.
Martin-Ortigosa S and Wang K. Proteolistics: a biolistic method for intracellular delivery of proteins. Transgenic Research 2014. 23: 743
McCormick S, Niedermeyer J, Fry J, Barnason A, Horsch R, Fraley R. (1986). Leaf disc transformation of cultivated tomato (L. esculentum) using Agrobacterium tumefaciens. Plant Cell Rep. 5(2):81-4.
Nagata T, Nemoto Y, Hasezawa S (1992) Tobacco BY-2 cell line as the "HeLa" cell in the cell biology of higher plants. International Review of Cytology 132: 1-30
Pospisilova J, Nada Wilhelmova, Helena Synkova, J. C atsky, D. Krebs, Ingrid Ticha, Richardson CD, Ray GJ, Bray NL, Corn JE. Non-homologous DNA increases gene disruption efficiency by altering DNA repair outcomes. Nat Commun. 2016;7:12463.
Rivera AL, Magaña-Ortíz D, Gómez-Lim M, Fernández F, Loske AM. Physical methods for genetic transformation of fungi and yeast. Physics of Life Reviews. 2014;11:184-203.
Sack M, Rademacher T, Spiegel H, Boes A, Hellwig S, Drossard J, Stoger E, Fischer R. (2015). From gene to harvest: insights into upstream process development for the GMP production of a monoclonal antibody in transgenic tobacco plants. Plant Biotechnol J. Oct; 13(8):1094-105.
Svitashev S, Schwartz C, Lenderts B, Young JK, Mark Cigan A. Genome editing in maize directed by CRISPR-Cas9 ribonucleoprotein complexes. Nat Commun. 2016; 7:13274.
Woo JW, Kim J, Kwon SI, Corvalán C, Cho SW, Kim H, Kim SG, Kim ST, Choe S1, Kim JS. DNA-free genome editing in plants with preassembled CRISPR-Cas9 ribonucleoproteins. Nature Biotechnology. 2015. 33(11):1162-4
Zhang B, Xia Yang, Chunping Yang, Mingyang Li and Yulong Guo. (2015). Exploiting the CRISPR/Cas9 System for Targeted Genome Mutagenesis in Petunia. Scientific Reports 6, Article number: 20315. doi:10.1038/srep20315.

## Claims

1. A method for altering the genome of an intact differentiated cell(s) without inserting exogenous genetic material into the genome comprising:
(i) providing an intact differentiated cell that comprises an endogenous gene to be modified;
(ii) providing a genome-modifying formulation comprising a pre-assembled ribonucleoprotein (RNP)-complex comprising a nucleic acid modifying protein and a ribonucleic acid;
(iii) delivering said RNP-complex into the cell by using a physical delivery method;
(iv) inducing one or more single or double stranded DNA breaks in the cell genome to produce a cell having a detectable targeted genomic modification without the presence of any exogenous genetic material in the cell genome.

2. The method according to claim 1, wherein the cell is obtained from a non-sterile environment and/or provided under a non-sterile environment.

3. The method according to any one of claims 1 to 2, wherein the differentiated cell(s) are exposed to stress conditions before and/or while delivering the pre-assembled ribonucleoprotein (RNP)-complex, including but not limited to UV-stress, heat-stress, cold-stress, draught, reactive oxygen-species or chemicals.

4. The method according to any one of claims 1 to 3, wherein the pre-assembled ribonucleoprotein (RNP)-complex is co-delivered with one or more components, including but not limited to dNTP, ddNTP, non-natural base analogues, di-nucleotides, trinucleotides, oligonucleotides , enzyme inhibitors, sugars, amino acids, proteins, antibodies, transcription factors and other DNA-binding proteins, ribonucleic acids in the form of mRNA, siRNA, miRNA.

5. The method according to any one of claims 1 to 4, wherein the genome-modifying formulation, in particular the (RNP)-complex, does not comprise DNA.

6. The method according to any one of claims 1 to 5, wherein the genome-modifying formulation, in particular the (RNP)-complex, does not comprise a marker gene.

7. The method according to any one of claims 1 to 6, wherein the genome-modifying formulation comprises a plurality of different pre-assembled ribonucleoprotein (RNP)-complex, in particular wherein the ribonucleoprotein (RNP)-complexes differ in the nucleic acid sequence of the ribonucleic acid comprised in the complexes.

8. The method according to any one of claims 1 to 7, wherein the differentiated cell is exposed to the pre-assembled ribonucleoprotein (RNP)-complex, in particular in the presence of a carrier material, wherein after delivering said RNP-complex into the cell, the genome of the cell is altered/modified, and wherein after the modification/alteration the cells are further cultivated to derive a cell, cell line, tissue or organism with a modified (altered) genome without the presence of a marker gene.

9. The method according to any one of claims 1 to 8, wherein the cell is a plant cell comprising a cell wall, in particular said plant cell is from a plant from the *Solanaceae* family, like *Nicotiana tabacum* and *Nicotiana benthamiana* from the genus *Nicotiana.*

10. The method according to any one of claims 1 to 9, wherein said intact plant cell is isolated from plant leaves, flowers, seeds, roots or cotyledons before providing the pre-assembled ribonucleoprotein (RNP)-complex.

11. The method according to any one of claims 1 to 10, wherein said delivery method is abrasion and/or perforation or wherein said delivery method is a biolistic particle delivery method.

12. The method according to any one of claims 1 to 11, wherein the pre-assembled RNP-complex is coated on a particle, in particular the pre-assembled RNP-complex is coated on the particle by air-drying, in particular the particle is a gold or tungsten particle.

13. The method according to any one of claims 1 to 12, wherein the nucleic acid modifying protein is a CRISPR-Cas9 endonuclease, a CRISPR-Cpf1 nuclease or a CRISPR-C2c2 endoribonuclease.

14. The method according to any one of claims 1 to 13, wherein said RNP-complex comprises a nucleic acid comprising a crRNA and a tracrRNA, or a chimeric cr/tracrRNA hybrid, wherein the crRNA and tracrRNA, or the cr/tracrRNA hybrid, is targeted to a sequence that is endogenous to the cell; and a Cas9 endonuclease molecule that induces a double strand break at or near the sequence to which the crRNA and tracrRNA sequence is targeted, or at or near the sequence to which the cr/tracrRNA hybrid is targeted.

15. The method according to any one of claims 1 to 14, wherein the cell is further cultivated in a non-sterile environment after said RNP-complex has been delivered.


SEQUENCE LISTING

<110>  Rheinisch-Westfälische Technische Hochschule (RWTH) Aachen

<120>  IMPROVED GENOME EDITING IN DIFFERENTIATED CELLS

<130>  TUA-PA02-EP

<160>  20

<170>  PatentIn version 3.5

<210>  1
<211>  23
<212>  DNA
<213>  Artificial Sequence

<220>
<223>  CRISPR/Cas9 recognition site in plasmid pUlli1250

<400>  1
gggagggaga gcttggcagg ggg                                              23


<210>  2
<211>  23
<212>  DNA
<213>  Nicotiana tabacum

<400>  2
gccgttaatt tgagagtcca agg                                              23


<210>  3
<211>  23
<212>  DNA
<213>  Nicotiana tabacum

<400>  3
tttggaatat caggtttggg tgg                                              23


<210>  4
<211>  23
<212>  DNA
<213>  Nicotiana tabacum

<400>  4
gaggcaagag atgtcctagg tgg                                              23


<210>  5
<211>  23
<212>  DNA
<213>  Nicotiana tabacum

<400>  5
gctgcatgga aagatgatga tgg                                              23


<210>  6
<211>  21
<212>  DNA

<213>  Artificial Sequence

<220>
<223>  Primer sequences used for amplifying the target region

<400>  6
   cttgattttg tgggtgaagg a                                                  21


<210>  7
<211>  20
<212>  DNA
<213>  Artificial Sequence

<220>
<223>  Primer sequences used for amplifying the target region

<400>  7
atggtttagt tgggcgtgag                                                   20


<210>  8
<211>  21
<212>  DNA
<213>  Artificial Sequence

<220>
<223>  Primer sequences used for amplifying the target regions

<400>  8
ctaaggcttg caatctgtga g                                                 21


<210>  9
<211>  27
<212>  DNA
<213>  Artificial Sequence

<220>
<223>  Primer sequences used for amplifying the target regions

<400>  9
ccatatcttt tacctaatat gctgtac                                           27


<210>  10
<211>  27
<212>  DNA
<213>  Artificial Sequence

<220>
<223>  Primer sequences used for amplifying the target region

<400>  10
gtacagcata ttaggtaaaa gatatgg                                           27


<210>  11
<211>  20
<212>  DNA
<213>  Artificial Sequence

<220>

<223>  Primer sequences used for amplifying the target region

<400>  11
gcaaggcaga atacagatcg                                                   20


<210>  12
<211>  23
<212>  DNA
<213>  Solanum lycopersicum

<400>  12
ggactcttgc cagcaatgct tgg                                               23


<210>  13
<211>  22
<212>  DNA
<213>  Artificial Sequence

<220>
<223>  Primer sequences for target region amplification Tomato

<400>  13
atggtgagct aatcacgagt aa                                                22


<210>  14
<211>  22
<212>  DNA
<213>  Artificial Sequence

<220>
<223>  Primer sequences for target region amplification tomato

<400>  14
aaagagaggc aggtagacaa tc                                                22


<210>  15
<211>  20
<212>  DNA
<213>  Petunia x hybrida

<400>  15
ggtgttcatt gccatgtcaa                                                   20


<210>  16
<211>  20
<212>  DNA
<213>  Petunia x hybrida

<400>  16
ggcatgcaaa gtctctcagg                                                   20


<210>  17
<211>  21
<212>  DNA
<213>  Artificial Sequence

<220>

<223>  Primer sequences for target region amplification

<400>  17
ccgaagctct tcctgctcca a                                                 21


<210>  18
<211>  24
<212>  DNA
<213>  Artificial Sequence

<220>
<223>  Primer sequences for target region amplification

<400>  18
ggtttctctg ggttgaggac atac                                              24


<210>  19
<211>  25
<212>  DNA
<213>  Artificial Sequence

<220>
<223>  Primer sequences for target region amplification

<400>  19
ggcgagatag gattctaacc tgaag                                             25


<210>  20
<211>  25
<212>  DNA
<213>  Artificial Sequence

<220>
<223>  Primer sequences for target region amplification

<400>  20
ggtactccga ctaacttctc caact                                             25